Europäisches Patentamt

(19) European Patent Office

Office européen des brevets

(11) Publication number: **0 024 505**
**B1**

(12) **EUROPEAN PATENT SPECIFICATION**

(45) Date of publication of patent specification: **20.10.82**

(51) Int. Cl.³: **C 07 C 69/80,**
**C 07 C 67/08, C 08 K 5/12**

(21) Application number: **80103672.4**

(22) Date of filing: **28.06.80**

(54) Method of making mixed esters of phthalic acid.

(30) Priority: **27.08.79 US 69636**

(43) Date of publication of application:
**11.03.81 Bulletin 81/10**

(45) Publication of the grant of the patent:
**20.10.82 Bulletin 82/42**

(84) Designated Contracting States:
**AT BE DE FR GB IT SE**

(56) References cited:
**DE - B - 1 225 164**
**US - A - 3 172 904**
**US - A - 3 825 515**
**US - A - 4 018 708**
**US - A - 4 020 010**

(73) Proprietor: **BASF WYANDOTTE CORPORATION**
**1609 Biddle Avenue**
**Wyandotte Michigan 48192 (US)**

(72) Inventor: **Bauer, William Herman, Jr.**
**8337 Colony Drive**
**Grosse Ile, Michigan 48138 (US)**

(74) Representative: **Michaelis, Wolfgang, Dr.**
**c/o BASF Aktiengesellschaft 38 Carl-Bosch-Strasse**
**D-6700 Ludwigshafen (DE)**

Courier Press, Leamington Spa, England.

# 0 024 505

## Method of making mixed esters of phthalic acid

This invention relates to a new method of preparing mixed esters of phthalic acid for use as plasticizers particularly for vinyl plastics and more specifically, mixed esters from phthalic anhydride, butyl alcohol, and octyl alcohol by sequential reaction.

A plasticizer has been defined as a material incorporated in a plastic to increase its workability and its flexibility and distensibility. The addition of the plasticizer may lower the melt viscosity, the temperature of the second-order transition, or the elastic modulus of the plastic. A class of such plasticizers, which has been widely used, is the phthalic acid esters of monoalcohols produced by condensing aliphatic monoalcohols containing not more than 20 carbon atoms with phthalic anhydride to form neutral products. Dioctylphthalate, known as DOP, which is most commonly bis-(2-ethylhexyl)-phthalate, is the industry standard for a general purpose plasticizer for poly (vinyl chloride) (PVC) and accounts for approximately one-fourth of the total plasticizer production. Since plasticization on poly (vinyl chloride) takes an estimated 80 percent of plasticizer production, most plasticizers are compared to DOP for price/performance relationships. The economic life of any other plasticizer depends upon its being less expensive than DOP or its doing some specific job better than DOP. The demand from the public in the last decade for high-quality products has resulted in continued growth of many different plasticizers with unique properties. Among the better known plasticizers for PVC are the co-esters of butanol and octanol with phthalic anhydride generally referred to as butyl octyl phthalate (BOP) which have been available in the U.S. market since the early 1950's and butyl benzyl phthalate (BBP). It must be understood that the products referred to on the market as butyl octyl phthalate are not necessarily completely butyl octyl phthalate. Generally, when made by the mixed ester route, the final product is a ternary mixture of dibutyl phthalate, butyl octyl phthalate and di-2-ethylhexyl phthalate. The ratio of these three components can vary widely, based not only upon the stoichiometry of the original reaction, but also upon the manufacturing procedure and catalyst employed. Generally, the prior art butyl octyl phthalate products tend to have around a 20/80 mole percent ratio of $C_4$ to $C_8$ carbon atom compounds.

During the 1950's, butyl octyl phthalate and butyl benzyl phthalate sold at a price of about one-half cent a pound below that of DOP. As such, these two plasticizers represented low-priced replacements for DOP. In the mid-1960's, both butyl octyl phthalate and butyl benzyl phthalate took the form of specialty plasticizers and the price rose above that of DOP. The specialty aspect emphasized was high solvency. This characteristic is particularly valuable in the manufacture of products such as vinyl asbestos tile, carpet backing, plastisols and organosols, coatings and as process aids. The latter, particularly, involving the addition of plasticizers to formulations used in calendering and extrusion where the primary performance characteristic is to reduce apparent melt viscosity at processing temperature and/or to provide improved surface gloss.

However, both products have the drawback of high volatility. In the case of the butyl octyl phthalate, this is due primarily to the presence of dibutyl phthalate. Accordingly, the purpose of the instant invention is to provide a higher solvating plasticizer than DOP with reduced volatility compared to BBP or prior art butyl octyl phthalate products by reducing the content of dibutyl phthalate (DBP) in butyl octyl phthalate.

U.S. Patent 3,172,904 discloses that, inter alia, mixed esters of dicarboxylic acids can be used as plasticizers, the said mixed esters being obtained by the stepwise esterification of dicarboxylic acid with two different alcohols. Since only unsatisfactory products are obtained in the production of these mixed esters using the method employed up to that time, in which the esterification with the first alcohol is taken to the half-ester is effected at a temperature of about 140—150°C in the absence of a catalyst, a process for overcoming these disadvantages is proposed in which the dicarboxylic acid is reacted with the first alcohol in the presence of an entrainer liquid and of phosphoric acid as catalyst and in which the esterification with the second alcohol is carried out at a temperature of between about 125° and 140°C in the presence of benzene sulfonic acid or another strong acid catalyst. This process is relatively complicated and expensive.

The present invention is directed to an improvement in the process for preparing butyl octyl phthalate products which are mixed esters of phthalic acid for use as plasticizers characterized by improving solvating characteristics and reduced volatility. In its preferred form, the mixed ester is made by first reacting a butyl alcohol with phthalic anhydride in a mole ratio of 0.2 to 1.0 mole of alcohol per mole of phthalic anhydride at a temperature of 90° to 130°C to provide a mixture containing the half-ester and unreacted phthalic anhydride. Octyl alcohol is then added in a stoichiometric amount based on the unreacted acid groups along with 10 to 50 percent of the stoichiometric amount of octyl alcohol as excess. An esterification catalyst is added and the reaction mixture heated at a temperature of 160° to 210°C for a time sufficient to carry the esterification to substantial completion. The unreacted acid is then neutralized, and the resulting ester product purified.

The present invention is directed to the process wherein a buty alcohol is reacted with phthalic anhydride in a mole ratio of 0.2 to 1.0 mole of alcohol per mole of phthalic anhydride at a temperature of 90° to 130°C to provide a mixture containing the half-ester and unreacted phthalic anhydride. The

2

butyl alcohols that may be employed include isobutyl alcohol and n-butyl alcohol. Reaction of the butanol with phthalic anhydride requires initial heating but exotherms near 90°C, and it is necessary to maintain the temperature below 130°C. The reaction generally requires 15 to 60 minutes. It is then cooled and the octyl alcohol is added along with a catalyst. The octyl alcohols that may be employed include isooctyl alcohol and 2-ethylhexyl alcohol. The mixture is heated with agitation to a temperature of 160°C at which point it refluxes. The temperature is allowed to rise slowly to a temperature of 200°C and is not permitted to exceed 210°C. The reaction is continued until the water ceases to separate in the distillate. When the acid number drops to 2—3, the reaction is considered complete. The reaction time generally runs from 5 to 25 hours depending on many factors such as the size of the reactor. When the reaction is complete, the mixture is cooled and the acid neutralized with an alkali such as sodium hydroxide in a sufficient amount determined by acid numbers to completely neutralize the acid plus 10 percent excess. The ester is washed by adding water and salt, stirred for 10 to 40 minutes, allowed to settle and the aqueous layer drawn off. It is then rewashed with water and salt mixture. The system is then closed and subjected to a vacuum from 38 to 64 cm of mercury absolute after which the alcohol is distilled off. After most of the alcohol is boiled off, the ester is stripped with steam until the free 2-ethylhexanol content is less than 150 parts per million. After stripping, the ester is further purified by adding powdered carbon, filter aid, attapulgus clay and filtering.

Suitable catalysts which may be employed in the second step of the reaction, i.e., the reaction of the half-ester prepared from the butanol and phthalic anhydride with the alkanol, include tetra-n-butyltitanate, a tin-titanate blend, and strong mineral acids such as sulfuric. Other catalysts that may be employed are p-toluenesulfonic acid, methanesulfonic acid, stannous oxalate, and orthophosphoric acid. Alkaline solutions which may be employed for neutralizing include aqueous sodium hydroxide, aqueous potassium hydroxide, sodium bicarbonate, and calcium hydroxide.

The following examples illustrate the invention. All parts are by weight and all temperatures are in degrees Centigrade unless otherwise indicated.

Example 1

A plurality of batches of mixed ester were prepared in a 113.6-liter glass-lined reactor, equipped with a multiple speed stirrer and a reflux condenser. The overhead system was set for total reflux with water flowing through the condenser. 10.4 kg of n-butanol (0.6 equivalent) was suctioned into the reactor followed by the addition of 34.5 kg of phthalic anhydride (1.0 equivlent). The system was then purged three times by drawing vacuum and adding nitrogen.

After this addition, the mixture was heated slowly with slow agitation, while being cautious to consider an exotherm near 90°C. The heated mixture was retained at 10±5°C with stirring for 15—20 minutes, and then cooled to 75±5°C. 51.7 kg (1.7 equivalence) 2-ethylhexyl alcohol was then suctioned into the reactor, and 0.03 kg tetrabutyl titanate catalyst was added. The system was then again purged three times by drawing vacuum and adding nitrogen. The overhead system was then adjusted for water separation.

The mixture was again heated with slow agitation and reflux began at about 160°C. The temperature was allowed to rise slowly from 160°C to 200°C, but not allowed to exceed 210°C. After four hours, the acid number was checked every hour. When the reaction was completed, 3.6 to 4 kg of water accumulated. A typical reaction time for the 2-ethylhexanol esterification was 15 hours. When the reaction was completed the mixture was cooled to 50±5°C and drawn off. This first crude batch had an acid number of 3.7, a butyl octyl phthalate to dioctyl phthalate ratio of 0.54 and the weight percent of dibutyl phthalate was 1.4 percent.

A second and a third crude batch was made according to the above procedure, with the second batch having an acid number of 2.8, and the third batch having an acid number of 1.8. The three crude batches were then charged into a 378.5 liter reactor and heated to 75±5°C. An amount of sodium hydroxide sufficient to neutralize the acid as determined by the acid numbers was added along with a 10 percent excess. The ester was then washed by adding 27.2 kg water and 1.4 kg of salt, stirred for 15 minutes, allowed to settle for 30 minutes, and the aqueous layer drawn off. The ester was then rewashed twice more with 56.8 kg water and 1.4 kg of salt with the mixture heated to 75±5°C. The acid number of the neutralized and washed ester mixture was less than 0.06.

The system was then closed and subjected to a vacuum of 50±13 cm of mercury absolute. The condenser water was turned on, and the solution heated to 145 ± 5°C to distill off alcohol. About 50 pounds of alcohol was expected. After most of the alcohol was boiled off, the ester was stripped with steam at the rate of 1.8 kg per hour for about 6 hours. The ester was sampled and stripping continued until the free 2-ethylhexanol content was less than 150 parts per million.

After stripping, the ester was further purified by adding 0.45 kg of powdered carbon, 0.45 kg of filter aid ([R] Celite), 0.45 kg of attapulgus clay, and filtered.

A second and third group of three batches were also prepared and worked up according to the procedure given above. The three groups containing the nine batches were then blended together to provide a mixed ester product.

Analyses of certain of the batches and blends were made during the preparation, and the results of these analyses are given in Table I below. In Table I, 2—EH represents 2-ethylhexanol, BOP

3

represents n-butyl, 2-ethylhexyl phthalate, DOP represents di-(2-ethylhexyl) phthalate, and DBP represents di-n-butyl phthalate.

TABLE I

Analysis of Esters

| Sample | Acid No. | ppm, 2—EH | ppm, $H_2O$ | BOP/DOP | Wt.% DBP |
|---|---|---|---|---|---|
| No. 1 Crude | 3.7 | — | — | 0.54 | 1.4 |
| No. 2 Crude | 2.8 | — | — | — | — |
| No. 3 Crude | 1.8 | — | — | — | — |
| 1—3 After carbon | 0.05 | <50 | 650 | 0.61 | 3.5 |
| No. 4 Crude | 2.1 | — | — | — | — |
| No. 5 Crude | 0.8 | — | — | — | — |
| No. 6 Crude | 1.0 | — | — | 0.39 | 1.0 |
| 4—6 After carbon | 0.005 | 350 | 200 | 0.44 | 2.7 |
| No. 7 Crude | 4.2 | — | — | 0.65 | 8.5 |
| No. 8 Crude | 2.8 | — | — | 0.64 | 5.5 |
| No. 9 Crude | 1.6 | — | — | 0.71 | 8.9 |
| 7—9 After carbon | 0.012 | <50 | 400 | 0.72 | 5.5 |
| Final Blend | 0.048 | 100 | 860 | 0.6 | 3.8 |

Table I shows that the final observed BOP/DOP ratio for crude ester varies from 0.39 to 0.72. In some batches, the percent DBP is also quite low. Laboratory batches, however, give a final observed ratio of *ca.* 0.7 and 3 to 4 percent DBP for the ester when 70/30 mole percent $C_8/C_4$ alcohols are used. These results suggest that butanol was lost from the system. Total material balance was low for batches 3—6 by 0.9 to 2.7 kg weight.

Esterifications were done at atmospheric pressure near 200°C maximum temperature with a mild nitrogen purge. Batches 3—6 were heated with more driving force for longer times, shown by the lower acid numbers of crude product. When small amounts of butanol were released into the system via transesterification, some of the butanol was undoubtedly carried through the condenser as a vapor. It is believed that using a higher excess of 2-ethylhexanol should also lower reaction boiling temperature which will minimize transesterification and subsequent loss of butanol.

However, the product obtained in Example 1 had excellent processing characteristics when used as a plasticizer for vinyl plastics.

Examples 2—8

A series of mixed esters were made in the laboratory in three different ranges of variation in the $C_4$ and $C_8$ alcohols used. Certain of the examples were made by coesterification (CE), and others were made by sequential addition (SA). The $C_4$ alcohol used was n-butyl alcohol (B), and the $C_8$ alcohol used was 2-ethylhexyl alcohol (O). In addition to these abbreviations, D is used in Table II below to designate di, P is used to designate phthalate, and MFT to designate minimum fluxing temperature. The minimum fluxing temperature is determined by the gradient hot bar method which is a bench test that can be effectively used to measure the static solvating characteristics of plasticizers. The apparatus basically consists of a 2.5 cm by 7.6 cm by 66 cm steel bar resting on a series of hot plates, the top side of which has a smooth finish. The heaters are adjusted to provide a gradient temperature on the surface of the bar over the range of approximately 40 to 200°C.

The minimum fluxing temperature is determined on a plastisol consisting of by weight 100 parts of poly (vinyl chloride) resin, 100 parts plasticizer, and 1.0 part stabilizer. In order to run the test, the

4

surface of the bar is lubricated lightly with silicone stopcock grease and wiped to leave an ultra-thin film. The plastisol is poured along the length of the bar in a uniform stream about one-quarter inch wide. After exactly 20 minutes, the fused end of the strip is lifted and is gently and steadily pulled away from the bar, maintaining the specimen at right angles to the bar. The strip will break at the point where fusion is not quite sufficient for cohesion to have developed. The temperature at this point is measured with a contact pyrometer and called the minimum fluxing temperature.

The sequential addition was carried out by first adding the $C_4$ alcohol to phthalic anhydride in the absence of catalyst and heating as described in Example 1 to make the half ester. Then the $C_8$ alcohol and catalyst (tetra-n-butyl titanate) was added and the reaction completed in the manner described in Example 1. In the coesterification reaction, both alcohols and the catalyst were added to the phthalic anhydride together. Analyses of the products and minimum fluxing temperatures are given in Table II below.

TABLE II

| Example | Mol % $C_4$ | Mol % $C_8$ | Method | DBP | BOP (Area %) | DOP | BOP/DOP | MFT |
|---|---|---|---|---|---|---|---|---|
| 2 | 20 | 80 | CE | 2.0 | 26.8 | 71.2 | 0.38 | 88.1 |
| 3 | 20 | 80 | CE | 2.2 | 27.1 | 70.7 | 0.38 | 89.8 |
| 4 | 20 | 80 | SA | 1.4 | 23.9 | 74.7 | 0.32 | 88.8 |
| 5 | 30 | 70 | SA | 4.2 | 37.6 | 58.2 | 0.65 | 83.8 |
| 6 | 30 | 70 | CE | 5.7 | 40.1 | 54.1 | 0.74 | 80.5 |
| 7 | 40 | 60 | SA | 7.9 | 48.8 | 43.2 | 1.13 | 74.4 |
| 8 | 40 | 60 | SA | 11.0 | 44.0 | 44.8 | 0.98 | 75.2 |

From the data given above, it is seen that sequential addition reduces the amount of dibutyl phthalate which results in reduced volatility, yet at the same time, the minimum fluxing temperatures are relatively close. Therefore, sequential addition provides an overall improvement in these combined properties.

Example 9

A series of sequential addition reactions were carried out using the procedure of Examples 4, 5 and 7 above, but with three different catalysts. The three catalysts studied were tetra-n-butyl titanate, a tin-titanate blend, and sulfuric acid. These studies showed no change in ester distribution with catalyst change. Therefore, the invention may be carried out with any of the usual esterification catalysts.

Example 10

In the data given for Examples 2—8, it was noted that the solvency improves (shown by MFT) as the ratio of n-buty, 2-ethylhexyl phthalate to di-2-ethylhexyl phthalate increases. In this example, a study was made to determine the effect of di-n-butyl phthalate on the solvency.

Therefore, di-n-butyl phthalate was added to the mixed ester of Example 3, and the minimum fluxing temperature measured. Table III below shows the data thus obtained.

TABLE III

| Mixed Exter | MFT |
|---|---|
| DOP | 95.6 |
| Example 3 | 89.8 |
| Example 3 + 5% DBP | 88.9 |
| Example 3 + 10% DBP | 88.9 |
| BBP | 65.0 |

From the data given in Table III, it is seen that the solvency is relatively unaffected by the DBP through the 0 to 10 percent range, even through lower DBP concentration reduces the problem caused by the volatility in the plasticizer.

Example 11

In this example, a plasticizer composition utilizing the invention was processed in a poly (vinyl chloride) formulation, and dynamic processing data was measured. Comparative results were also obtained by processing two conventional plasticizers in the same manner. The plasticizer of the invention was a 50—50 mix of the plasticizers of Examples 7 and 8 and is indicated in Table IV below as BOP. The composition of the 50—50 mix is as follows:

| Weight Percent | | Weight Ratio | |
|---|---|---|---|
| DBP | BOP | DOP | BOP/DOP |
| 7.8 | 46.7 | 45.5 | 1.03 |

The conventional plasticizers were di-2-ethylhexyl phthalate (DOP) and butyl benzyl phthalate (BBP).

The recipe for the formulations were PVC GP—4 type, 100 parts by weight; plasticizer, 40 parts by weight; limestone, 20 parts by weight; and Ba-Cd solid, 3 parts by weight. The dynamic process characteristics of these formulations were evaluated on a Brabender Plasti-Corder, and the data are given in Table IV below.

TABLE IV

| Plasticizer | Time to Flux | Temperature at Flux | Peak Torque | Torque 15' After Peak Torque | Temperature 15' After Peak Torque |
|---|---|---|---|---|---|
| BOP | 1.5' | 140°C | 2500 M-gms | 1820 M-gms | 158°C |
| DOP | 2.5' | 146°C | 2150 M-gms | 1640 M-gms | 161°C |
| BBP | 1.5' | 140°C | 2900 M-gms | 1770 M-gms | 158°C |

Claims

1. A method of making a mixed ester plasticizer for vinyl plastics and the like comprising the steps of first reacting a butyl alcohol with phthalic anhydride in a mole ratio of 0.2 to 1.0 mole of alcohol per mole of phthalic anhydride at a temperature of 90° to 130°C to provide a mixture containing a half-ester and unreacted phthalic anhydride, adding an octyl alcohol in a stoichiometric amount based on unreacted acid groups along with 10 to 50 percent of the stoichiometric amount of octyl alcohol as excess, adding an esterification catalyst and heating the reaction mixture at a temperature of 160°C to 210°C for a time sufficient to carry the esterification to substantial completion.

2. The method of claim 1 which includes the additional steps of neutralizing the unreacted acid with an aqueous alkaline solution capable of converting the acid to water-soluble salts, and purifying the resultant ester mixture by the removal of salt solution, excess alkaline solution, excess alcohol and catalyst.

3. The method of claim 1 wherein said butyl alcohol is n-butyl alcohol and said octyl alcohol is 2-ethylhexyl alcohol.

4. The method of claim 3 including the additional steps of neutralizing the unreacted acid with an aqueous alkaline solution capable of converting the acid to water-soluble salts, and purifying the resultant ester mixture by removal of salt solution, excess alkaline solution, excess alcohol and catalyst.

. 5. The method of claim 4 wherein said esterification catalyst is tetra-n-butyltitanate.

6. The method of claim 4 wherein said esterification catalyst is a tin-titanate blend.

7. The method of claim 4 wherein said esterification catalyst is a strong mineral acid.

8. The process of claim 4 wherein said reaction of butyl alcohol with phthalic anhydride is at a temperature of 90 to 130°C for a period of time of 15 to 60 minutes and the subsequent reaction with said octyl alcohol is at a temperature of 160 to 210°C for a time of from 5 to 25 hours.

**Revendications**

1. Procédé pour préparer un plastifiant mixte de type ester pour matières plastiques vinyliques et similaires, caractérisé par le fait qu'on fait d'abord réagir un alcool butylique avec l'anhydride phtalique, dans un rapport molaire de 0,2 à 1,0 mole d'alcool par mole d'anhydride phtalique, à une température de 90 à 130°C, pour produire un mélange contenant un hémi-ester et de l'anhydride phtalique n'ayant pas réagi, on ajoute un alcool octylique en quantité stoechiométrique par rapport aux groupes acides n'ayant pas réagi, aved 10 à 50% de la quantité stoechiométrique d'alcool octylique comme excès, on ajoute un catalyseur d'estérification et on chauffe le mélange de réaction à une température de 160 à 210°C pendant une durée suffisante pour amener pratiquement l'estérification à son achèvement.

2. Procédé selon la revendication 1, dans lequel, en outre, on neutralise l'acide n'ayant pas réagi, avec une solution aqueuse alcaline capable de transformer l'acide en sels solubles dans l'eau et on purifie le mélange d'esters obtenu par èlimination de la solution de sels, de l'excès de solution alcaline, de l'excès d'alcool et du catalyseur.

3. Procédé selon la revendication 1, dans lequel ledit alcool butylique est l'alcool n-butylique et ledit alcool octylique est l'alcool éthyl-2 hexylique.

4. Procédé selon la revendication 3, dans lequel, en outre, on neutralise l'acide n'ayant pas réagi avec une solution alcaline aqueuse capable de transformer l'acide en sels solubles dans l'eau et on purifie le mélange d'esters obtenu par élimination de la solution de sels, de l'excès de la solution alcaline, de l'excès d'alcool et du catalyseur.

5. Procédé selon la revendication 4, dans lequel ledit catalyseur d'estérification est le titanate de tétra-n-butyle.

6. Procédé selon la revendication 4 dans lequel ledit catalyseur d'estérification est un mélange étain-titanate.

7. Procédé selon la revendication 4, dans lequel ledit catalyseur d'estérification est un acide minéral fort.

8. Procédé selon la revendication 4, dans lequel ladite réaction de l'alcool butylique avec l'anhydride phtalique est effectuée à une témpérature de 90 à 130°C, pendant une période de 15 à 60 minutes, puis la réaction avec ledit alcool octylique est effectuée à une température de 160 à 210°C, pendant une durée de 5 à 25 heures.

**Patentansprüche**

1. Verfahren zur Herstellung eines Misc ester-Weichmachers für Vinylkunststoffe usw., dadurch gekennzeichnet, daß man zunächst einen Butylalkohol mit Phthalsäureanhydrid in einem Molverhältnis von 0,2 bis 1,0 Mol Alkohol pro Mol Phthalsäureanhydrid bei einer Temperatur von 90 bis 130°C zu einer Mischung umsetzt, die einen Halbester und nichtumgesetztes Phthalsäureanhydrid enthält, dann einen Oktylalkohol in stöchiometrischer Menge, bezogen auf nichtumgesetzte Säuregruppen, zusammen mit einem Überschuß an Oktylalkohol von 10 bis 50% der stöchiometrischen Menge zugibt, einen Veresterungskatalysator hinzufügt und das Reaktionsgemisch bis zur im wesentlichen vollständigen Veresterung bei 160 bis 210°C erhitzt.

2. Verfahren nach Anspruch 1, dadurch gekennzeichnet, daß man in zusätzlichen Verfahrensschritten die nichtumgesetzte Säure mit einer wäßrigen alkalischen Lösung, die die Säure in wasserlösliche Salze zu überführen vermag, neutralisiert und die erhaltene Estermischung durch Abtrennen der Salzlösung, der überschüssigen alkalischen Lösung, der überschüssigen Alkohols und des Katalysators reinigt.

3. Verfahren nach Anspruch 1, dadurch gekennzeichnet, daß man also Butylalkohol n-Butylalkohol und als Oktylalkohol 2-Ethylhexylalkohol verwendet.

4. Verfahren nach Anspruch 3, dadurch gekennzeichnet, daß man in zusätzlichen Verfahrensschritten die nichtumgesetzte Säure mit einer wäßrigen alkalischen Lösung, die die Säure in wasserlösliche Salz zu überführen vermag, neutralisiert und die erhaltene Estermischung durch Abtrennen der Salzlösung, der überschüssigen alkalischen Lösung, des überschüssigen Alkohols und des Katalysators reinigt.

**0 024 505**

5. Verfahren nach Anspruch 4, dadurch gekennzeichnet, daß man als Veresterungskatalysator Tetra-n-butyltitanat verwendet.

6. Verfahren nach Anspruch 4, dadurch gekennzeichnet, daß man als Veresterungskatalysator eine Zinn-Titanat-Mischung verwendet.

7. Verfahren nach Anspruch 4, dadurch gekennzeichnet, daß man als Veresterungskatalysator eine starke Mineralsäure verwendet.

8. Verfahren nach Anspruch 4, dadurch gekennzeichnet, daß man die Umsetzung von Butylalkohol mit Phthalsäureanhydrid 15 bis 60 Minuten lang bei 90 bis 130°C und die nachfolgende Umsetzung mit Oktylalkohol 5 bis 25 Stunden lang bei 160 bis 210°C durchführt.